# EUROPÄISCHE PATENTSCHRIFT

(11) **EP 3 761 921 B1**
(45) Veröffentlichungstag und Bekanntmachung des Hinweises auf die Patenterteilung: **20.04.2022**
(21) Anmeldenummer: 19710373.2
(22) Anmeldetag: 07.03.2019
(51) Int. Cl.: A61F 5/01

(54) **ORTHESE MIT WERKZEUGLOS FESTLEGBAREM DECKEL**
ORTHOSIS COMPRISING A CAP THAT CAN BE FIXED WITHOUT THE USE OF TOOLS
ORTHÈSE POURVUE D'UN COUVERCLE POUVANT ÊTRE FIXÉ SANS OUTILS

(30) Priorität: 09.03.2018 DE 102018105482
(43) Veröffentlichungstag der Anmeldung: 13.01.2021
(73) Patentinhaber: Albrecht GmbH, 83071 Stephanskirchen (DE)
(72) Erfinder: OPAHLE, Hans-Georg, 83024 Rosenheim (DE)
(74) Vertreter: Flach Bauer Stahl Patentanwälte Partnerschaft mbB
(86) Internationale Anmeldenummer: PCT/EP2019/055696
(87) Internationale Veröffentlichungsnummer: WO 2019/170804

(56) Entgegenhaltungen:
- US-A- 5 399 154
- US-A- 5 520 627
- US-A1- 2014 308 065
- US-A1- 2016 151 190

## Beschreibung

Die Erfindung betrifft eine Orthese mit einem ersten und zweiten Schienenarm, die in einem Gelenkabschnitt gelenkig miteinander verbunden und relativ zueinander um eine Schwenkachse schwenkbar sind, gemäß dem Oberbegriff des Anspruches 1.

Orthopädische Orthesen werden bekannterweise dafür verwendet, Gelenke wie beispielsweise Knie- oder Ellbogengelenke zu führen und zu stabilisieren und/oder Gliedmaßen mittels Federkraft in Extensions- oder Flexionsrichtung zu drängen.

Derartige Orthesen weisen bekanntermaßen ein Achselement auf, das unmittelbar oder mittelbar an einem der Schienenarme befestigt ist. Der zweite Schienenarm ist schwenkbar am Achselement gelagert. Weiterhin weisen bekannte Orthesen dieser Art in der Regel einen Deckel auf, mit dem eine Seite des Gelenkabschnitts überwiegend oder vollständig abgedeckt ist. Die Befestigung eines Deckels erfolgt üblicherweise mittels mindestens einer Schraube, die in eine entsprechende Gewindebohrung im Gelenkabschnitt der Orthese mittels eines entsprechenden Werkzeugs eingeschraubt wird.

Nachteilig ist hierbei, dass zum Befestigen und Lösen des Deckels am bzw. vom Gelenkabschnitt ein Werkzeug benötigt wird, das nicht immer zur Verfügung steht. Das Entfernen des Deckels kann beispielsweise immer dann erforderlich sein, wenn unter dem Deckel liegende Schwenkbereichanschlagstifte, die den maximalen Schwenkbereich der Schienenarme relativ zueinander begrenzen, verstellt werden sollen, um den maximalen Schwenkbereich zu verändern.

Aus der US 2016/0151190 A1 ist eine Orthese gemäß dem Oberbegriff des Anspruchs 1 bekannt. Dort wird ein Bajonettverschluss verwendet, um ein Aufnahmeelement, bei dem es sich beispielsweise um ein Federelement oder einen Anschlag handeln kann, in einer Bohrung eines Gelenkgrundkörpers zu befestigen.

Aus der US 2014/0308065 A1 ist eine Orthese bekannt, bei der eine Krafterzeugungseinheit drehfest auf ein zentrales Achselement aufgesteckt werden kann, wobei die Krafterzeugungseinheit mittels eines Rasthakens verriegelt wird.

Der Erfindung liegt die Aufgabe zugrunde, eine Orthese der eingangs genannten Art zu schaffen, bei welcher der Deckel auf möglichst einfache Weise und werkzeuglos am Gelenkabschnitt befestigt und von diesem gelöst werden kann.

Diese Aufgabe wird erfindungsgemäß durch eine Orthese mit den Merkmalen des Anspruches 1 gelöst. Vorteilhafte Ausführungsformen der Erfindung sind in den weiteren Ansprüchen beschrieben.

Bei der erfindungsgemäßen Orthese weist das Achselement einen Halsabschnitt und einen daran angrenzenden Kopfabschnitt mit mindestens einem radial über den Halsabschnitt vorragenden Radialvorsprung auf, der einen ersten Teil der bajonettartigen Haltevorrichtung bildet. Weiterhin weist der Deckel eine mittige Deckelöffnung zum Hindurchführen des Kopfabschnitts und mindestens einen Verriegelungswandabschnitt auf, der einen zweiten Teil der bajonettartigen Haltevorrichtung bildet, in der ersten Drehposition neben dem Radialvorsprung angeordnet ist und in der zweiten Drehposition den Radialvorsprung untergreift.

Mittels der erfindungsgemäßen Orthese kann der Deckel auf sehr einfache, schnelle und werkzeuglose Weise am Gelenkabschnitt der Orthese befestigt und von diesem gelöst werden. Zur Befestigung des Deckels muss dieser lediglich axial auf das Achselement aufgesteckt und anschließend in eine bestimmte Richtung gedreht werden. Zum Lösen des Deckels ist es lediglich erforderlich, diesen in umgekehrter Richtung zu drehen und anschließend axial vom Achselement abzunehmen. Der Deckel kann dabei ohne weiteres derart ausgebildet sein, dass er eine Abdeckung für darunter angeordnete Schwenkbereichanschlagstifte bildet, so dass der Deckel einerseits einen sehr schnellen und einfachen Zugang zu den Schwenkbereichanschlagstiften ermöglicht, wenn diese verstellt werden sollen, und andererseits die Schwenkbereichanschlagstifte auf sehr schnelle und einfache Weise abdeckt und gegen ein Herausfallen aus den entsprechenden Einsteckbohrungen verhindert.

Vorzugsweise weist der Radialvorsprung eine Umfangswand mit kreisbogenförmiger Außenkontur auf, wobei der Deckel eine zur Deckelöffnung benachbarte Vertiefung mit kreisförmigen Umfangswandabschnitten aufweist, an denen der Deckel radial am Achselement geführt ist. Der Radialvorsprung dient in diesem Fall nicht nur zur axialen Verriegelung, sondern auch zur radialen Zentrierung des Deckels am Achselement. Alternativ ist es auch möglich, den Deckel auf andere Weise radial zu zentrieren, beispielsweise indem der Verriegelungswandabschnitt des Deckels mit seiner inneren Umfangswand am Halsabschnitt des Achselements anliegt.

Vorzugsweise ist der Verriegelungswandabschnitt des Deckels derart vertieft im Deckel angeordnet, dass der Kopfabschnitt des Achselements axial nach außen nicht über den Deckel übersteht. Hierdurch kann eine geringe Bauhöhe im Bereich des Gelenkabschnitts erzielt und die außenliegende Stirnfläche des Gelenkabschnitts ohne vorstehende Teile ausgebildet werden.

Vorzugsweise weist der Kopfabschnitt eine ebene Stirnfläche auf, die in der gleichen Ebene wie die umgebende Deckelwand liegt.

Gemäß einer besonders vorteilhaften Ausführungsform weist das Achselement zwei diametral gegenüberliegende Radialvorsprünge auf, während der Deckel zwei diametral gegenüberliegende Verriegelungswandabschnitte aufweist. Hierdurch wird der Deckel besonders gleichmäßig diametral am Achselement festgelegt. Besonders zweckmäßig ist es dabei, wenn die beiden gegenüberliegenden Radialvorsprünge des Achselements die gleiche Form und Größe haben. Dasselbe gilt für die beiden gegenüberliegenden Verriegelungswandabschnitte des Deckels. Weiterhin ist es auch ohne weiteres möglich, eine andere Anzahl von Radialvorsprüngen und Verriegelungswandabschnitten vorzusehen, insbesondere drei oder vier Radialvorsprünge und Verriegelungswandabschnitte.

Vorteilhafterweise erstrecken sich die Radialvorsprünge des Achselements in Umfangsrichtung jeweils über einen Winkelbereich von 85° bis 95°, insbesondere etwa 90°. In diesem Fall erstrecken sich die Verriegelungswandabschnitte des Deckels in Umfangsrichtung ebenfalls jeweils über einen Winkelbereich von 85° bis 95°, insbesondere etwa 90°.

Vorzugsweise weist der Deckel ein bogenförmiges Langloch auf, wobei die Orthese einen relativ zum ersten Schienenarm stationären Anschlagstift aufweist, der sich durch das Langloch hindurch erstreckt und den Drehbereich des Deckels begrenzt. Zweckmäßigerweise sind dabei der Anschlagstift und das Langloch derart relativ zur bajonettartigen Haltevorrichtung positioniert, dass der Anschlagstift in der ersten Drehposition des Deckels, in welcher der Deckel auf das Achselement aufgesteckt werden kann, an einem Ende des Langlochs angeordnet ist. Dies schafft eine einfache Positionierungshilfe beim Aufsetzen des Deckels auf den Gelenkabschnitt und ermöglicht darüber hinaus das schnelle und genaue Positionieren des Deckels, wenn dieser von seiner verriegelten zweiten Drehposition in die erste Drehposition zurückgedreht werden soll, um den Deckel vom Gelenkabschnitt abzuheben.

Vorzugsweise weist die Orthese eine Rasteinrichtung zum Verrasten des Deckels in seiner zweiten Drehposition auf, wobei die Rasteinrichtung ein federndes Rastelement, das am ersten Schienenarm gehaltert ist, und eine Rastvertiefung umfasst, die am Deckel derart angeordnet ist, dass das Rastelement in der zweiten Drehposition des Deckels in die Rastvertiefung verrastend eingreift. Hierdurch kann auf sehr einfache Weise sichergestellt werden, dass der Deckel in seiner Verriegelungsposition (zweite Drehposition) bleibt und sich nicht selbständig in seine Löseposition (erste Drehposition) zurückdrehen kann.

Alternativ hierzu ist es auch möglich, dass das federnde Rastelement nicht am ersten Schienenarm, sondern am Deckel gehaltert ist, während die Rastvertiefung an einem relativ zum ersten Schienenarm stationär angeordneten Teil angeordnet ist.

Die Erfindung wird nachfolgend anhand der Zeichnungen beispielhaft näher erläutert. Es zeigen:
- Figur 1:: eine räumliche Darstellung der erfindungsgemäßen Orthese mit verkürzt dargestellten Schienenarmen;
- Figur 2:: eine Explosionsdarstellung der erfindungsgemäßen Orthese;
- Figur 3:: eine Draufsicht auf einen Anschlagstifthalter;
- Figur 4:: eine Seitenansicht eines Anschlagstifts;
- Figur 5:: eine Draufsicht auf den Anschlagstift von Figur 4;
- Figur 6:: eine räumliche Darstellung des Achselements;
- Figur 7:: einen Längsschnitt durch das Achselement von Figur 6;
- Figur 8:: eine Ansicht des Deckels von unten;
- Figur 9:: einen Schnitt durch den Deckel längs der Linie IX-IX von Figur 10;
- Figur 10:: eine Draufsicht auf den Deckel;
- Figur 11:: Teile der Rasteinrichtung in Explosionsdarstellung; und
- Figur 12:: eine Darstellung des Gelenkbereichs der erfindungsgemäßen Orthese ohne Deckel, wobei das Achselement geschnitten dargestellt ist.

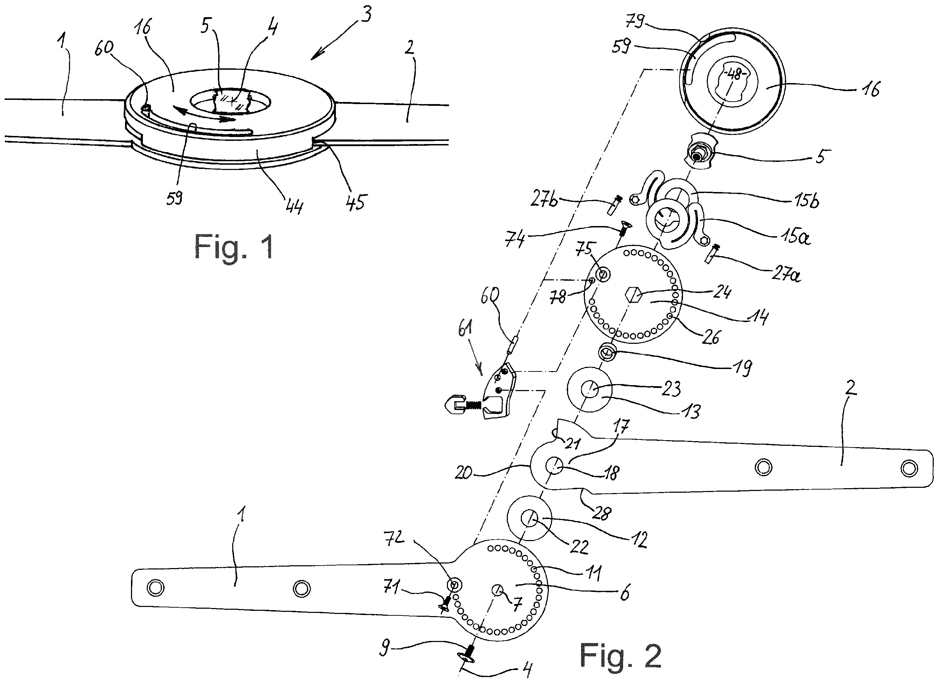

Figur 1 zeigt einen mittleren Gelenkabschnitt 3 einer erfindungsgemäßen Orthese mit einem ersten Schienenarm 1 und einem zweiten Schienenarm 2. Die beiden Schienenarme 1, 2 sind lediglich verkürzt dargestellt und weisen nicht dargestellte Befestigungseinrichtungen, insbesondere Klettbänder, Schalen oder dergleichen, auf, mit denen die Schienenarme 1, 2 in bekannter Weise an mittels eines Körpergelenks verbundenen Gliedmaßen, beispielsweise am Ober- und Unterschenkel, Ober- und Unterarm etc., befestigt werden können.

Die Schienenarme 1, 2 sind in einem Gelenkabschnitt 3 gelenkig miteinander verbunden und um eine Schwenkachse 4 relativ zueinander schwenkbar. Die Schwenkachse 4 wird durch ein Achselement 5 gebildet, das am ersten Schienenarm 1 festgelegt ist.

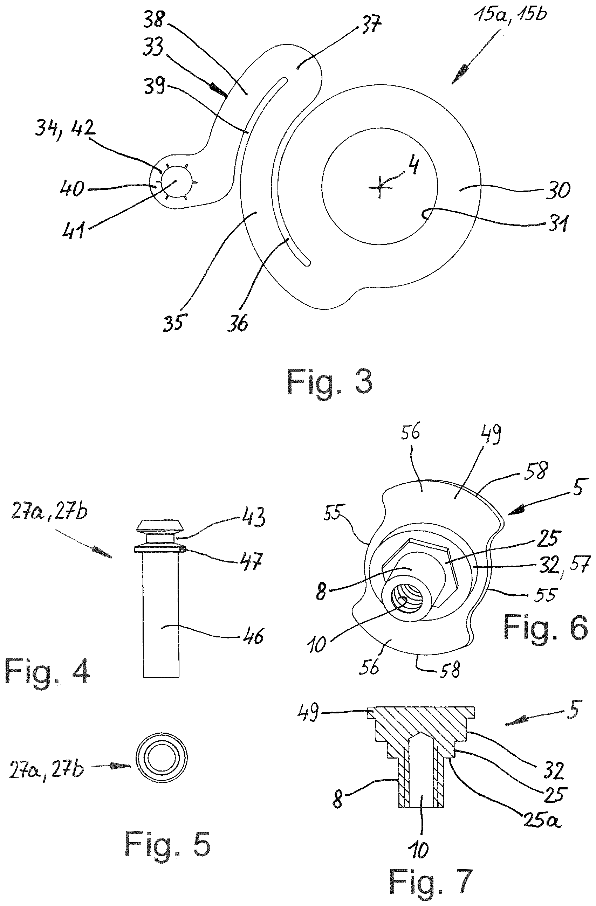

Figur 2 zeigt in Explosionsdarstellung den Aufbau der erfindungsgemäßen Orthese. Der erste Schienenarm 1 weist einen kreisscheibenförmigen Endbereich 6 mit einer zentralen Bohrung 7 auf. Diese Bohrung 7 dient zur Aufnahme eines zylinderförmigen Abschnitts 8 (Figuren 6 und 7) des Achselements 5 und zum Hindurchführen einer zentralen Schraube 9, die in eine axiale Gewindebohrung 10 des Achselements 5 eingeschraubt werden kann, um das Achselement 5 und den ersten Schienenarm 1 drehfest miteinander zu verbinden.

In einem randnahen Bereich des kreisscheibenförmigen Endbereichs 6 ist eine Mehrzahl von Schwenkbereichsbegrenzungslöchern 11 vorgesehen, die auf einem zur Schwenkachse 4 konzentrischen Kreisbogen angeordnet sind.

In Axialrichtung aufeinanderfolgend weist die Orthese ferner eine erste Reibungsverminderungsscheibe 12, den zweiten Schienenarm 2, eine zweite Reibungsverminderungsscheibe 13, eine Lochscheibe 14, zwei Anschlagstifthalter 15a, 15b, das Achselement 5 und einen Deckel 16 auf. Es wird darauf hingewiesen, dass der in den Figuren 2 und 8 bis 10 gezeigte Deckel 16 ein "rechter" Deckel ist, während Figur 1 eine Orthese mit einem "linken" Deckel 16 zeigt, der sich in der Position eines Langlochs 59 in Umfangsrichtung relativ zur Ausrichtung einer Deckelöffnung 48 von einem "rechten" Deckel 16 geringfügig unterscheidet, im Übrigen jedoch gleich ausgebildet ist.

Die beiden Reibungsverminderungsscheiben 12, 13 bestehen zweckmäßigerweise aus Kunststoff, insbesondere Teflon.

Der zweite Schienenarm 2 weist einen Endbereich 17 mit einer Bohrung 18 auf. Die Bohrung 18 dient zur Aufnahme einer zylindrischen Lagerhülse 19, die auf dem zylindrischen Abschnitt 8 des Achselements 5 drehbar gelagert ist, so dass der zweite Schienenarm 2 um die Schwenkachse 4 herum relativ zum ersten Schienenarm 1 verschwenkbar ist.

Der Endbereich 17 des zweiten Schienenarms 2 weist über etwa 180° einen kreisbogenförmigen Umfang 20 auf. An diesen kreisbogenförmigen Umfang 20 schließt sich eine radial vorstehende Anschlagfläche 21 an.

Die beiden Reibungsverminderungsscheiben 12, 13 weisen zentrale Löcher 22, 23 auf, die in gleicher Weise wie die Bohrung 18 zum Hindurchführen der Lagerhülse 19 dienen.

Die Lochscheibe 14 ist als Kreisscheibe ausgebildet und parallel zum Endbereich 6 des ersten Schienenarms 1 in einem derartigen feststehenden Abstand angeordnet, dass der zweite Schienenarm 2 dazwischen verschwenkt werden kann. Dieser Abstand wird durch die Länge der zylindrischen Lagerhülse 19 bestimmt, die mit einer Stirnseite an einer von einem Mehrkantabschnitt 25 des Achselements 5 gebildeten Durchmesserstufe 25a und mit ihrer anderen Stirnseite am ersten Schienenarm 1 anliegt. Die Lochscheibe 14 weist eine mittige Öffnung 24 mit einer mehreckigen Kontur auf, die zum drehfesten Aufstecken auf den entsprechend ausgebildeten Mehrkantabschnitt 25 des Achselements 5 dient.

In einem randnahen Bereich der Lochscheibe 14 sind eine Mehrzahl von Schwenkbereichbegrenzungslöchern 26 vorgesehen, die zu den Schwenkbereichbegrenzungslöchern 11 des ersten Schienenarms 1 fluchten und wie diese auf einem zur Schwenkachse 4 konzentrischen Kreisbogen angeordnet sind. Die Schwenkbereichbegrenzungslöcher 11, 26 erstrecken sich im gezeigten Ausführungsbeispiel längs eines Kreisbogens über etwa 270° und können beispielsweise derart angeordnet sein, dass der Winkelabstand benachbarter Schwenkbereichbegrenzungslöcher 11, 26 jeweils 10° beträgt.

Die Schwenkbereichbegrenzungslöcher 11, 26 dienen zum Einstecken von zwei Anschlagstiften 27a, 27b zur Begrenzung des Schwenkbereichs der Orthese in Extensions- und Flexionsrichtung. Die Anschlagstifte 27a, 27b weisen hierzu eine zylindrischen Einsteckabschnitt 46 (Figur 4) auf, der parallel zur Schwenkachse 4 in ein fluchtendes Paar von Schwenkbereichbegrenzungslöchern 11, 26 bis zu einem radial vorspringenden Kragen 47 eingesteckt werden kann. Die Schwenkbereichsbegrenzung in Extensionsrichtung wird bewirkt, indem die Anschlagfläche 21 des zweiten Schienenarms 2 an einem der Anschlagstifte 27a, 27b anschlägt. Die Schwenkbereichsbegrenzung in Flexionsrichtung wird bewirkt, indem eine Anschlagfläche 28 des zweiten Schienenarms 2 am anderen Anschlagstift 27b, 27a anschlägt. Durch Einstecken der Anschlagstifte 27a, 27b in unterschiedliche Schwenkbereichbegrenzungslöcher 11, 26 können somit die Schwenkbereichsgrenzen der Orthese sowohl in Extensions- als auch in Flexionsrichtung verändert werden.

Die Anschlagstifte 27a, 27b werden jeweils durch einen separaten Anschlagstifthalter 15a, 15b unverlierbar am Achselement 5 gehalten. Im dargestellten Ausführungsbeispiel sind die beiden Anschlagstifthalter 15a, 15b gleich ausgebildet, jedoch spiegelbildlich gedreht am Achselement 5 angeordnet. Zweckmäßigerweise sind die beiden Anschlagstifthalter 15a, 15b in Axialrichtung der Orthese benachbart angeordnet.

Die Ausgestaltung der Anschlagstifthalter 15a, 15b geht aus Figur 3 hervor. Jeder Anschlagstifthalter 15a, 15b besteht aus einer ebenen, dünnen Blattfeder aus Federstahlblech, dessen Dicke zweckmäßigerweise etwa 0,2 bis 0,5 mm, insbesondere 0,3 mm, beträgt. Andere Materialien und Dicken sind möglich.

Weiterhin umfasst jeder Anschlagstifthalter 15a, 15b einen ringförmigen Lagerabschnitt 30 mit einer mittigen Öffnung 31 zum Hindurchführen eines zylinderförmigen Achsabschnitts 32 des Achselements 5 (Figuren 6 und 7), auf dem der Anschlagstifthalter 15a, 15b drehbar gelagert ist. Die beiden Anschlagstifthalter 15a, 15b sind somit unabhängig voneinander um die Schwenkachse 4 drehbar.

Mit dem ringförmigen Lagerabschnitt 30 ist ein federnder, elastisch biegsamer Haltearm 33 verbunden, an dessen freiem Ende eine Stiftbefestigungseinrichtung 34 zur Befestigung des Anschlagstifts 27a, 27b vorgesehen ist.

Der Haltearm 33 umfasst einen ersten Armabschnitt 35, der konzentrisch zur Schwenkachse 4 in einer ersten Umfangsrichtung über einen Winkelbereich von etwa 110° verläuft. Der erste Armabschnitt 35 kann andere Längen aufweisen und sich beispielsweise über einen Winkelbereich von 30-180° erstrecken. Weiterhin ist der erste Armabschnitt 35 vom Lagerabschnitt 30 durch einen Spalt 36 getrennt, dessen Breite geringer als die Breite des ersten Armabschnitts 35 ist.

Der erste Armabschnitt 35 geht mittels eines 180°-Bogenabschnitts 37 in einen zweiten Armabschnitt 38 über, der ebenfalls konzentrisch zur Schwenkachse 4, jedoch in einer zweiten Richtung verläuft, die zur ersten Richtung gegenläufig ist. Die Breite des Spalts 39 zwischen dem zweiten Armabschnitt 38 und dem ersten Armabschnitt 35 ist wiederum geringer als die Breite der Armabschnitte 35, 38. Weiterhin erstreckt sich der zweite Armabschnitt 38 im gezeigten Ausführungsbeispiel über einen Winkelbereich von etwa 45-55°. Auch hier ist die Länge des zweiten Armabschnitts 38 in großem Umfang variabel.

Durch den mäanderförmigen Verlauf des Haltearms 33 kann dieser bei sehr kleiner Baugröße eine große Länge aufweisen. Hierdurch kann das freie Ende 4 des Haltearms 33, an dem der Anschlagstift 27a, 27b befestigt ist, ausreichend weit senkrecht zur Hauptebene des Anschlagstifthalters 15a, 15b hochgehoben werden, um den Anschlagstift 27a, 27b vollkommen aus den Schwenkbereichbegrenzungslöchern 11, 26 herausziehen und in einem gewünschten Schwenkbereichbegrenzungsloch 11, 26 einsetzen zu können. Da das Herausziehen der Anschlagstifte 27a, 27b aus den Schwenkbereichbegrenzungslöchern 11, 26 nur gegen die Federkraft der Anschlagstifthalter 15a, 15b möglich ist, verhindern die Anschlagstifthalter 15a, 15b gleichzeitig eine unerwünschte Axialverschiebung oder ein Lösen der Anschlagstifte 27a, 27b aus den Schwenkbereichbegrenzungslöchern 11, 26.

Die am freien Ende 40 des Haltearms 33 angeordnete Stiftbefestigungseinrichtung 34 ist im dargestellten Ausführungsbeispiel als lösbare Befestigungseinrichtung in Form einer Clipverbindung zur Befestigung des Anschlagstifts 27a, 27b ausgebildet. Hierzu weist die Stiftbefestigungseinrichtung 34 ein Einsteckloch 41 zum Einstecken des Anschlagstifts 27a, 27b und Feder- oder Rastzungen 42 auf, die in eine Umfangsnut 43 des Anschlagstifts 27a, 27b (Figur 4) eingreifen und dadurch den Anschlagstift 27a, 27b sowohl radial als auch axial halten. Wie in Figur 3 dargestellt, erstrecken sich die Feder- oder Rastzungen 42 um den gesamten Umfang des Einstecklochs 41 herum nach innen und können durch randseitige radiale Einschnitte in das Federstahlblech gebildet werden.

Wie aus den Figuren 1 und 8 bis 10 ersichtlich, ist beim dargestellten Ausführungsbeispiel der teller- bzw. napfförmige und in der Draufsicht kreisförmige Deckel 16 derart ausgebildet, dass sich seine Umfangswand 44 über die Lochscheibe 14 und teilweise bis nahe zum ersten Schienenarm 1 erstreckt. Eine seitliche Aussparung 45 in der Umfangswand 44 schafft dabei den notwendigen Freiraum zum Verschwenken des zweiten Schienenarms 2 über einen maximalen Schwenkbereich von beispielsweise 120-160°, insbesondere von etwa 140° relativ zum ersten Schienenarm 1. Dieser maximale Schwenkbereich kann jedoch in weitem Umfang variieren.

Der Deckel 16 weist eine Stirnwand 46 mit einer ebenen, ringförmig umlaufenden Außenfläche 47 auf. Eine mittige Deckelöffnung 48 weist eine Form auf, die an die Außenkontur eines Kopfabschnitts 49 (Figuren 6 und 7) des Achselements 5 derart angepasst ist, dass dieser Kopfabschnitt 49 durch die Deckelöffnung 48 hindurchgesteckt bzw. der Deckel 16 auf den Kopfabschnitt 49 aufgesteckt werden kann, wobei sich der Kopfabschnitt 49 in die Deckelöffnung 48 hineinerstreckt.

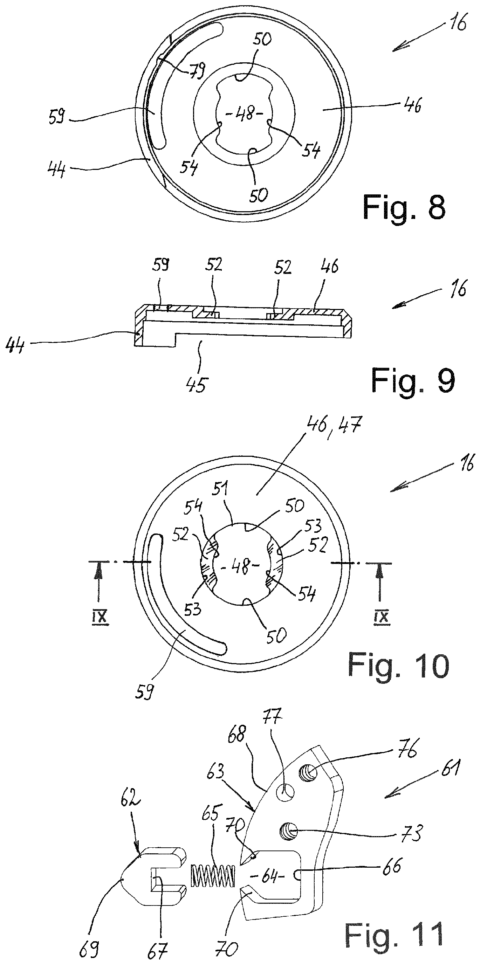

Wie aus Figur 10 ersichtlich, ist die Deckelöffnung 48 durch zwei diametral gegenüberliegende Umfangswandabschnitte 50 begrenzt, die sich in Umfangsrichtung jeweils über etwa 90° erstrecken. Die Umfangswandabschnitte 50 liegen in der in Figur 10 dargestellten Draufsicht auf einem Kreis 51. Weiterhin wird die Deckelöffnung 48 durch zwei diametral gegenüberliegende seitliche Verriegelungswandabschnitte 52 begrenzt, die sich vom Kreis 51 nach innen erstrecken und relativ zur Außenfläche 47 vertieft in der Stirnwand 56 angeordnet sind. Die Umfangswandabschnitte 50 sind dabei durch Umfangswandabschnitte 53 miteinander verbunden, die in der in Figur 10 gezeigten Draufsicht ebenfalls auf dem Kreis 51 liegen, jedoch eine geringere Höhe haben, da sie axial nach innen durch die Verriegelungswandabschnitte 52 begrenzt werden. Weiterhin weisen die Verriegelungswandabschnitte 52 jeweils eine Innenumfangswand 54 auf, deren Form an die seitliche Außenkontur 55 (Figur 6) des Kopfabschnitts 49 des Achselements 5 angepasst ist, so dass der Kopfabschnitt 49 mit lediglich geringem seitlichen Spiel durch die Deckelöffnung 48 hindurchgeführt werden kann, bis die Verriegelungswandabschnitte 52 des Deckels 16 unterhalb des Kopfabschnitts 49 des Achselements 5 liegen.

Der Deckel 16 und das Achselement 5 sind somit derart ausgestaltet, dass der Deckel 16 mittels einer bajonettartigen Haltevorrichtung, die einerseits durch den Kopfabschnitt 49 des Achselements 5 und andererseits durch die Verriegelungswandabschnitte 52 des Deckels 16 gebildet wird, am Achselement 5 festlegbar ist. Hierzu weist, wie aus Figur 6 ersichtlich, der Kopfabschnitt 49 des Achselements 5 zwei diametral gegenüberliegende Radialvorsprünge 56 auf, die radial über einen durch den zylindrischen Achsabschnitt 32 gebildeten Halsabschnitt 57 vorstehen und dazu dienen, die Verriegelungswandabschnitte 52 des Deckels 16 in einer bestimmten Drehstellung des Deckels 16 zu übergreifen, um den Deckel 16 axial in einer Richtung zu verriegeln. Die Radialvorsprünge 56 weisen jeweils eine kreisbogenförmige Umfangswand 58 auf, die bei aufgesetztem Deckel 16 an den inneren Umfangswandabschnitten 50, 53 des Deckels 16 anliegen oder mit nur geringem Spiel zu diesem angeordnet sind, so dass der Deckel 16 durch die Umfangswand 58 des Achselements 5 radial geführt wird.

Aufgrund der beschriebenen Ausgestaltung kann der Deckel 16 in einer ersten Drehposition, in der die Radialvorsprünge 56 des Achselements 5 den Umfangswandabschnitten 50 des Deckels 16 gegenüberliegen, auf das Achselement 5 aufgesteckt werden, bis die Oberseite der Verriegelungswandabschnitte 52 in einer Ebene liegt, die unterhalb der Unterseite des Kopfabschnitts 49 liegt. Wird anschließend der Deckel 16 um die Schwenkachse 4 in eine zweite Drehposition gedreht, die unterschiedlich ist zu derjenigen der ersten Drehposition, gelangen die Verriegelungswandabschnitte 52 des Deckels 16 in den Bereich unterhalb der Radialvorsprünge 56 des Achselements 5, wodurch der Deckel 16 gegen axiales Abheben von der Orthese bzw. vom Achselement 5 gesichert ist.

Im gezeigten Ausführungsbeispiel kann der Deckel 16 um etwa 90° von seiner das Aufsetzen und Entfernen des Deckels 16 ermöglichenden ersten Drehposition bis hin zu seiner verriegelten Endposition (= weite Drehposition) gedreht werden.

Im gezeigten Ausführungsbeispiel sind die Verriegelungswandabschnitte 52 des Deckels 16 relativ zu dessen Außenfläche 47 um ein Maß vertieft angeordnet, das der Dicke des Kopfabschnitts 49 des Achselements 5 entspricht. Hierdurch wird erreicht, dass die äußere Kopfabschnitt-Stirnfläche, die zweckmäßigerweise eben ausgebildet ist, bei aufgesetztem Deckel 16 nicht über die Außenfläche 47 des Deckels 16 vorsteht und zweckmäßigerweise in der gleichen Ebene wie die umgebende Deckelwand, d.h. wie die Außenfläche 47, liegt. Der Kopfabschnitt 49 des Achselements 5 geht somit auf seiner Außenseite stufenlos in die umgebende Außenfläche 47 des Deckels 16 über.

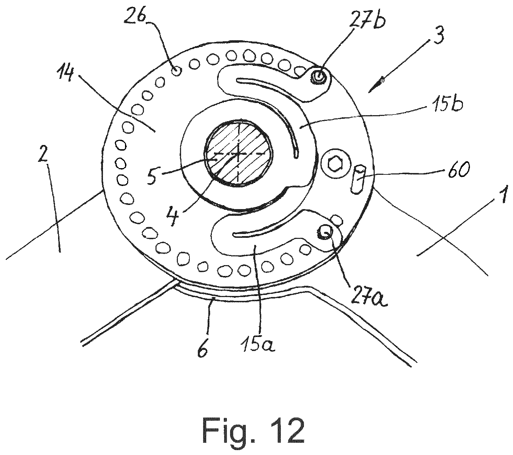

Um die erste Drehposition zum Aufsetzen und Entfernen des Deckels 16 auf einfache Weise zu finden und den Drehbereich des Deckels 16 zwischen dieser ersten Drehposition und der zweiten Dreh- oder Verriegelungsposition zu begrenzen, weist der Deckel 16 ferner ein kreisbogenförmiges Langloch 59 auf. Dieses Langloch 59 ist zweckmäßigerweise in der Stirnwand 46 in der Nähe des äußeren Randes des Deckels 16 angeordnet. Im gezeigten Ausführungsbeispiel erstreckt sich das Langloch 59 über einen Winkelbereich von etwa 90°, beispielsweise über einen Winkelbereich von 85° bis 95°. Durch dieses Langloch 59 erstreckt sich ein Anschlagstift 60 (Figuren 1 und 12) hindurch, der relativ zum ersten Schienenarm 1 stationär an der Orthese befestigt ist. In der ersten Drehposition befindet sich der Anschlagstift 60 an einem Ende des Langlochs 59. Beim Drehen des Deckels 16 von dieser ersten Drehposition in seine Verriegelungsstellung gleitet der Anschlagstift 60 innerhalb des Langlochs 59, bis der Anschlagstift 60 am gegenüberliegenden Ende des Langlochs 59 anschlägt und dadurch ein Weiterdrehen des Deckels 16 blockiert.

In der zweiten Drehposition, in welcher der Deckel 16 verriegelt ist, wird der Deckel 16 durch eine Rasteinrichtung 61 gehalten, die in Explosionsdarstellung in den Figuren 2 und 11 dargestellt ist.

Die Rasteinrichtung 61 umfasst ein Rastelement 62 und einen plattenförmigen Rastelementhalter 63. Das Rastelement 62 ist in einer Aussparung 64 innerhalb des Rastelementhalters 63 aufgenommen und wird durch eine Feder 65 radial nach außen, d.h. in Figur 11 nach links, gedrückt. Die Feder 65 stützt sich dabei mit einem Ende an der rückseitigen Wandfläche 66 und mit ihrem gegenüberliegenden Ende an einer Wandfläche 67 des Rastelements 62 ab. Die Aussparung 64 ist radial nach außen, d.h. zur kreisbogenförmigen Umfangswand 68 des Rastelementhalters 63 hin, offen, so dass der vordere Endabschnitt 69 des Rastelements 62, der im gezeigten Ausführungsbeispiel keilförmig ausgebildet ist, radial nach außen über die Umfangswand 68 vorragen kann. Durch die Feder 65, die als Druckfeder ausgebildet ist, wird das Rastelement 62 fortwährend in diese vorragende Position gedrängt. Die vorragende Endposition des Rastelements 62 wird dabei durch schräg aufeinander zulaufende Anschlagwände 70 des Rastelementhalters 63 begrenzt. Weiterhin kann sich das Rastelement 62 innerhalb der Aussparung 64 soweit entgegen der Kraft der Feder 65 radial nach innen zurückbewegen, dass das Rastelement 62 nicht mehr über die Umfangswand 68 des Rastelementhalters 63 vorsteht.

Die Rasteinrichtung 61 wird zwischen dem ersten Schienenarm 1 und der Lochscheibe 14 mittels einer Schraube 71 (Figur 2) am ersten Schienenarm 1 festgeschraubt, indem die Schraube 71 durch eine entsprechende Bohrung 72 des ersten Schienenarms 1 hindurchgeführt und in eine Gewindebohrung 73 (Figur 11) im Rastelementhalter 63 eingeschraubt wird. Die Rastvorrichtung 61 befindet sich dabei in der gleichen Ebene, jedoch radial außerhalb des Endbereichs 17 des zweiten Schienenarms 2. Eine weitere Schraube 74 (Figur 2) wird von der gegenüberliegenden Seite her durch eine Bohrung 75 in der Lochscheibe 14 hindurchgeführt und in eine Gewindebohrung 76 (Figur 11) im Rastelementhalter 63 eingeschraubt. Mittels des Rastelementhalters 63 ist somit die Lochscheibe 14 drehfest mit dem ersten Schienenarm 1 verbunden. Weiterhin dient der Rastelementhalter 63 zum Befestigen des Anschlagstifts 60, der in eine Bohrung 77 des Rastelementhalters 63 eingesetzt und durch eine Bohrung 78 in der Lochscheibe 14 hindurchgeführt ist.

Der vordere Endbereich 69 des Rastelements 62 wirkt mit einer Rastvertiefung 79 (Figur 8) zusammen, die an der Innenseite der Umfangswand 44 des Deckels 16 angeordnet ist. Diese Rastvertiefung 79 ist derart positioniert, dass der vordere Endabschnitt 69 des Rastelements 62 in die Rastvertiefung 79 verrastend eingreift, wenn sich der Deckel 16 in seiner verriegelten Endposition, d.h. in der zweiten Drehposition, befindet. Zum Zurückdrehen des Deckels 16 in die erste Drehposition ist es somit erforderlich, dass ein erhöhter Drehwiderstand überwunden werden muss, um das Rastelement 62 entgegen der Druckkraft der Feder 65 radial etwas nach innen zu drücken. Hierdurch wird der Deckel 16 in seiner verriegelten Endposition gesichert.

Im Rahmen der Erfindung ist eine Vielzahl von Variationen möglich. Beispielsweise ist es möglich, das federnde Rastelement 62 und die Rastvertiefung 79 kinematisch umzukehren, d.h. das Rastelement am Deckel 16 und die Rastvertiefung 79 an einem relativ zum ersten Schienenarm 1 stationär angeordneten Teil anzuordnen. Weiterhin kann die bajonettartige Haltevorrichtung zum Festlegen des Deckels 16 am Achselement 5 bei unterschiedlichsten Orthesen eingesetzt werden, beispielsweise auch bei Orthesen, die zum Quengeln in Extensions- und/oder Flexionsrichtung bestimmt sind.

Die erfindungsgemäße Orthese weist den besonderen Vorteil auf, dass der Deckel 16 auf werkzeuglose, schnelle und einfache Weise aufgesetzt und gelöst werden kann. Zusätzlich verhindert der Deckel 16, dass die Anschlagstifte 27a, 27b zur Begrenzung des Schwenkbereichs aus den Schwenkbereichbegrenzungslöchern 11, 26 herausfallen können, da die Anschlagstifte 27a, 27b von der Stirnwand 46 des Deckels 16 überdeckt werden.

## Patentansprüche

1. Orthese mit einem ersten und zweiten Schienenarm (1, 2), die in einem Gelenkabschnitt (3) gelenkig miteinander verbunden und relativ zueinander um eine Schwenkachse (4) schwenkbar sind, mit einem am ersten Schienenarm (1) unmittelbar oder mittelbar befestigten Achselement (5), an dem der zweite Schienenarm (2) schwenkbar gelagert ist, und mit einem Deckel (16) zur zumindest teilweisen Abdeckung des Gelenkabschnitts (3), wobei der Deckel (16) mittels einer bajonettartigen Haltevorrichtung am Achselement (5) festlegbar ist, und wobei der Deckel (16) in einer ersten Drehposition auf das Achselement (5) aufsetzbar und durch Drehen in eine zweite Drehposition am Achselement (5) axial verriegelbar ist, **dadurch gekennzeichnet, dass** das Achselement (5) einen Halsabschnitt (57) und einen daran angrenzenden Kopfabschnitt (49) mit mindestens einem radial über den Halsabschnitt (57) vorragenden Radialvorsprung (56) aufweist, der einen ersten Teil der bajonettartigen Haltevorrichtung bildet, und dass der Deckel (16) eine mittige Deckelöffnung (48) zum Hindurchführen des Kopfabschnitts (49) und mindestens einen Verriegelungswandabschnitt (52) aufweist, der einen zweiten Teil der bajonettartigen Haltevorrichtung bildet, in der ersten Drehposition neben dem Radialvorsprung (56) angeordnet ist und in der zweiten Drehposition den Radialvorsprung (56) untergreift.

2. Orthese nach Anspruch 1, **dadurch gekennzeichnet, dass** der Radialvorsprung (56) eine Umfangswand (58) mit kreisbogenförmiger Außenkontur aufweist, und dass der Deckel (16) eine zur Deckelöffnung (48) benachbarte Vertiefung mit kreisförmigen Umfangswandabschnitten (50, 53) aufweist, an denen der Deckel (16) radial am Achselement (5) geführt ist.

3. Orthese nach einem der vorhergehenden Ansprüche, **dadurch gekennzeichnet, dass** der Verriegelungswandabschnitt (52) des Deckels (16) derart vertieft im Deckel (16) angeordnet ist, dass der Kopfabschnitt (49) des Achselements (5) axial nach außen nicht über den Deckel (16) übersteht.

4. Orthese nach einem der vorhergehenden Ansprüche, **dadurch gekennzeichnet, dass** der Kopfabschnitt (49) eine ebene Stirnfläche aufweist, die in der gleichen Ebene wie die umgebende Deckelwand liegt.

5. Orthese nach einem der vorhergehenden Ansprüche, **dadurch gekennzeichnet, dass** das Achselement (5) zwei diametral gegenüberliegende Radialvorsprünge (56) aufweist, und dass der Deckel (16) zwei diametral gegenüberliegende Verriegelungswandabschnitte (52) aufweist.

6. Orthese nach Anspruch 5, **dadurch gekennzeichnet, dass** sich die Radialvorsprünge (56) in Umfangsrichtung jeweils über einen Winkelbereich von 85° bis 95° erstrecken.

7. Orthese nach einem der vorhergehenden Ansprüche, **dadurch gekennzeichnet, dass** der Deckel (16) ein bogenförmiges Langloch (59) zur Begrenzung des Drehbereichs aufweist, und dass die Orthese einen relativ zum ersten Schienenarm (1) stationären Anschlagstift (60) aufweist, der sich durch das Langloch (59) hindurch erstreckt und den Drehbereich des Deckels (16) begrenzt.

8. Orthese nach einem der vorhergehenden Ansprüche, **dadurch gekennzeichnet, dass** die Orthese eine Rasteinrichtung (61) zum Verrasten des Deckels (16) in seiner zweiten Drehposition aufweist, wobei die Rasteinrichtung (61) ein federndes Rastelement (62), das mit dem ersten Schienenarm (1) gekoppelt ist, und eine Rastvertiefung (79) umfasst, die am Deckel (16) derart angeordnet ist, dass das Rastelement (62) in der zweiten Drehposition des Deckels (16) in die Rastvertiefung (79) verrastend eingreift.

9. Orthese nach einem der Ansprüche 1 bis 7, **dadurch gekennzeichnet, dass** die Orthese eine Rasteinrichtung (61) zum Verrasten des Deckels (16) in seiner zweiten Drehposition aufweist, wobei die Rasteinrichtung (61) ein federndes Rastelement (62), das am Deckel (16) gehaltert ist, und eine Rastvertiefung (79) umfasst, die an einem relativ zum ersten Schienenarm (1) stationär angeordneten Teil derart angeordnet ist, dass das Rastelement (62) in der zweiten Drehposition des Deckels (16) in die Rastvertiefung (79) verrastend eingreift.

## Claims

1. Orthosis comprising a first and second brace arm (1, 2) which are interconnected in an articulated manner in a joint portion (3) and can be pivoted relative to one another about a pivot axis (4), a shaft element (5) which is directly or indirectly fastened to the first brace arm (1) and on which the second brace arm (2) is pivotably mounted, and a cover (16) for at least partially covering the joint portion (3), wherein the cover (16) can be fixed to the shaft element (5) by means of a bayonet-like holding apparatus, and wherein the cover (16) can be placed on the shaft element (5) in a first rotational position and can be axially locked on the shaft element (5) by rotation into a second rotational position, **characterised in that** the shaft element (5) has a neck portion (57) and a head portion (49) adjacent thereto having at least one radial projection (56) which projects radially beyond the neck portion (57) and forms a first part of the bayonet-like holding apparatus, and **in that** the cover (16) has a central cover opening (48) for passing through the head portion (49) and at least one locking wall portion (52), which forms a second part of the bayonet-like holding apparatus, is arranged next to the radial projection (56) in the first rotational position and engages under the radial projection (56) in the second rotational position.

2. Orthosis according to claim 1, **characterised in that** the radial projection (56) has a peripheral wall (58) having an outer contour in the shape of a circular arc, and **in that** the cover (16) has a recess which is adjacent to the cover opening (48) and has circular peripheral wall portions (50, 53) on which the cover (16) is guided radially on the shaft element (5).

3. Orthosis according to either of the preceding claims, **characterised in that** the locking wall portion (52) of the cover (16) is arranged so as to be recessed in the cover (16) in such a way that the head portion (49) of the shaft element (5) does not project axially outwards beyond the cover (16).

4. Orthosis according to any of the preceding claims, **characterised in that** the head portion (49) has a planar end face which lies in the same plane as the surrounding cover wall.

5. Orthosis according to any of the preceding claims, **characterised in that** the shaft element (5) has two diametrically opposite radial projections (56), and **in that** the cover (16) has two diametrically opposite locking wall portions (52).

6. Orthosis according to claim 5, **characterised in that** the radial projections (56) each extend in the peripheral direction over an angular range of 85° to 95°.

7. Orthosis according to any of the preceding claims, **characterised in that** the cover (16) has a curved elongate hole (59) for limiting the range of rotation, and **in that** the orthosis has a stop pin (60) which is stationary relative to the first brace arm (1), and which extends through the elongate hole (59) and limits the range of rotation of the cover (16).

8. Orthosis according to any of the preceding claims, **characterised in that** the orthosis has a latching device (61) for latching the cover (16) in the second rotational position thereof, wherein the latching device (61) comprises a resilient latching element (62) which is coupled to the first brace arm (1) and a latching recess (79) which is arranged on the cover (16) in such a way that the latching element (62) engages in the latching recess (79) in a latching manner in the second rotational position of the cover (16).

9. Orthosis according to any of claims 1 to 7, **characterised in that** the orthosis has a latching device (61) for latching the cover (16) in the second rotational position thereof, wherein the latching device (61) comprises a resilient latching element (62) which is held on the cover (16) and a latching recess (79) which is arranged on a part arranged in a stationary manner relative to the first brace arm (1) in such a way that the latching element (62) engages in the latching recess (79) in a latching manner in the second rotational position of the cover (16).

## Revendications

1. Orthèse pourvue d'une première et d'une deuxième branche d'attelle (1, 2), qui sont reliées ensemble de manière articulée dans une partie d'articulation (3) et sont pivotables l'une par rapport à l'autre autour d'un axe de pivotement (4), et pourvue d'un élément d'axe (5) fixé immédiatement ou non sur la première branche d'attelle (1), sur lequel la deuxième branche d'attelle (2) est logée en pouvant pivoter, et pourvue d'un couvercle (16) permettant le recouvrement au moins partiel de la partie d'articulation (3), où le couvercle (16) peut être fixé sur l'élément d'axe (5) au moyen d'un dispositif de maintien de type baïonnette, et où le couvercle (16) peut être placé sur l'élément d'axe (5) dans une première position de rotation et peut être verrouillé axialement par rotation sur l'élément d'axe (5) dans une deuxième position de rotation, **caractérisée en ce que** l'élément d'axe (5) présente une partie en collerette (57) et une partie de tête (49) y étant adjacente avec au moins une protubérance radiale (56) faisant saillie radialement par-dessus la partie en collerette (57), qui forme une première partie du dispositif de maintien de type baïonnette, et que le couvercle (16) présente un orifice de couvercle (48) central pour le passage de la partie de tête (49) et au moins une partie latérale de verrouillage (52), qui forme une deuxième partie du dispositif de maintien de type baïonnette, qui, dans la première position de rotation, est disposée à côté de la protubérance radiale (56) et se met en prise en-dessous de la protubérance radiale (56) dans la deuxième position de rotation.

2. Orthèse selon la revendication 1, **caractérisée en ce que** la protubérance radiale (56) présente une paroi périphérique (58) avec un contour extérieur en forme d'arc de cercle et que le couvercle (16) présente une cavité voisine par rapport à l'orifice de couvercle (48) avec des segments de paroi périphérique (50, 53) en forme de cercles, sur lesquels le couvercle (16) est guidé radialement sur l'élément d'axe (5).

3. Orthèse selon l'une des revendications précédentes, **caractérisée en ce que** le segment de paroi de verrouillage (52) du couvercle (16) est disposé en profondeur dans le couvercle (16) de telle manière que la partie de tête (49) de l'élément d'axe (5) ne fait pas saillie axialement vers l'extérieur par-dessus le couvercle (16) .

4. Orthèse selon l'une des revendications précédentes, **caractérisée en ce que** la partie de tête (49) présente une surface frontale qui se situe dans le même plan que la paroi de couvercle périphérique.

5. Orthèse selon l'une des revendications précédentes, **caractérisée en ce que** l'élément d'axe (5) présente deux protubérances radiales (56) opposées diamétralement et que le couvercle (16) présente deux parties de paroi de verrouillage (52) opposées diamétralement.

6. Orthèse selon la revendication 5, **caractérisée en ce que** les protubérances radiales (56) s'étendent dans la direction circonférentielle respectivement sur une plage angulaire de 85 ° à 95 °.

7. Orthèse selon l'une des revendications précédentes, **caractérisée en ce que** le couvercle (16) présente un trou oblong (59) en forme d'arc pour la délimitation de la zone de rotation et que l'orthèse présente une goupille de butée (60) stationnaire par rapport à la première branche d'attelle (1), qui s'étend à travers le trou oblong (59) et délimite la zone de rotation du couvercle (16).

8. Orthèse selon l'une des revendications précédentes, **caractérisée en ce que** l'orthèse présente un dispositif de verrouillage (61) pour le verrouillage du couvercle (16) dans sa deuxième position de rotation, où le dispositif de verrouillage (61) comprend un élément de verrouillage (62) élastique, qui est couplé avec la première branche d'attelle (1), et une cavité de verrouillage (79), qui est disposée sur le couvercle (16) de telle manière que l'élément de verrouillage (62) se met en prise en se verrouillant dans la cavité de verrouillage (79) dans la deuxième position de rotation du couvercle (16).

9. Orthèse selon l'une des revendications 1 à 7, **caractérisée en ce que** l'orthèse présente un dispositif de verrouillage (61) permettant de verrouiller le couvercle (16) dans sa deuxième position de rotation, où le dispositif de verrouillage (61) comprend un élément de verrouillage (62) élastique, qui est maintenu sur le couvercle (16), et une cavité de verrouillage (79), qui est disposée sur une partie disposée de manière stationnaire par rapport à la première branche d'attelle (1), de telle manière que l'élément de verrouillage (62) se met en prise en se verrouillant dans la cavité de verrouillage (79) dans la deuxième position de rotation du couvercle (16).
